# FASCICULE DE BREVET EUROPEEN

(11) **EP 3 164 140 B1**
(45) Date de publication et mention de la délivrance du brevet: **25.09.2019**
(21) Numéro de dépôt: 15713912.2
(22) Date de dépôt: 08.04.2015
(51) Int. Cl.: A61K 35/748, A61K 33/00, A61K 36/82, A61K 31/352, A61K 8/9706, A61K 8/25, A61K 36/87, A61K 36/9068, A61Q 19/00, A61K 31/695, A61K 36/53, A61K 36/63, A23L 29/00, A23L 17/60

(54) **MICROALGUE ENRICHIE EN SILICIUM SOUS UNE FORME SOLUBLE DANS L'EAU**
MIT SILIZIUM ANGEREICHERTE MIKROALGEN IN EINER WASSERLÖSLICHEN FORM
MICROALGA ENRICHED WITH SILICON IN A WATER-SOLUBLE FORM

(30) Priorité: 08.04.2014 FR 1453113
(43) Date de publication de la demande: 10.05.2017
(73) Titulaire: Phyco- Biotech, 34070 Montpellier (FR)
(72) Inventeur: BACCOU, Jean-Claude, F-34080 Montpellier (FR); GAY, Gilbert, F-34070 Montpellier (FR); JOUY, Nicolas, F-34980 St Gely du Fesc (FR)
(74) Mandataire: Argyma
(86) Numéro de dépôt international: PCT/EP2015/057596
(87) Numéro de publication internationale: WO 2015/155224

(56) Documents cités:
- US-A1- 2013 205 850
- Phycobiotech: "Solution articulations | Phycobiotech", , 2013, XP055138443, Extrait de l'Internet: URL:http://www.phyco-biotech.com/fr/soluti on-articulations [extrait le 2014-09-05]
- Phycobiotech: "Solution articulations | Phycobiotech", , 2013, XP055138444, Extrait de l'Internet: URL:http://www.phyco-biotech.com/fr/soluti on-articulations [extrait le 2014-09-05]
- DATABASE WPI Week 201415 Thomson Scientific, London, GB; AN 2014-D61538 XP002729467, & CN 103 462 043 A (WANG P) 25 décembre 2013 (2013-12-25)
- DATABASE WPI Week 201372 Thomson Scientific, London, GB; AN 2013-R40008 XP002729468, & CN 103 125 808 A (ZU T) 5 juin 2013 (2013-06-05) & CN 103 125 808 A (ZU TINGXUN) 5 juin 2013 (2013-06-05)
- LELA MUNJAS JURKIC ET AL: "Biological and therapeutic effects of ortho-silicic acid and some ortho-silicic acid-releasing compounds: New perspectives for therapy", NUTRITION & METABOLISM, BIOMED CENTRAL. LONDON, GB, vol. 10, no. 1, 8 janvier 2013 (2013-01-08), page 2, XP021137105, ISSN: 1743-7075, DOI: 10.1186/1743-7075-10-2
- MARTIN K R: "The chemistry of silica and its potential health benefits", JOURNAL OF NUTRITION, HEALTH AND AGING,, vol. 11, no. 2, 1 mars 2007 (2007-03-01), pages 94-97, XP009180011, ISSN: 1279-7707
- MARTIN KEITH R: "Silicon: the health benefits of a metalloid", METAL IONS IN LIFE SCIENCES, WILEY, UK, vol. 13, 1 janvier 2013 (2013-01-01), pages 451-473, XP009180010, ISSN: 1559-0836, DOI: 10.1007/978-94-007-7500-8_14
- PUYFOULHOUX GREGOIRE ET AL: "Iron availability from iron-fortified spirulina by an in vitro digestion/Caco-2 cell culture model", JOURNAL OF AGRICULTURAL AND FOOD CHEMISTRY, AMERICAN CHEMICAL SOCIETY, US, vol. 49, no. 3, 1 mars 2001 (2001-03-01), pages 1625-1629, XP002511446, ISSN: 0021-8561, DOI: 10.1021/JF001193C [extrait le 2001-03-01]

## Description

La présente invention a pour objet une composition comprenant une microalgue enrichie en silicium présent sous une forme soluble dans l'eau, et un procédé permettant la préparation d'une telle composition. L'invention a également pour objet une composition pharmaceutique, une composition alimentaire ou une composition cosmétique comprenant une microalgue enrichie en silicium sous une forme soluble dans l'eau, selon l'invention. Selon un aspect particulier de l'invention, ladite microalgue est la spiruline.

Le silicium est un oligoélément présent en faible quantité dans le corps humain. Cependant, il a été démontré l'importance de son rôle dans la formation des os en améliorant l'absorption du calcium, la formation du collagène, composant essentiel de l'architecture de la peau et des tissus conjonctifs notamment de la paroi des artères et des cartilages et le bon état des ongles. Le silicium se situe principalement dans le tissu conjonctif où il agit comme un agent de réticulation. On le retrouve dans les macromolécules comme les glycosaminoglycanes, le collagène et l'élastine (Seaborne C.D. *et al.,* 1993). Des taux élevés de silicium liés sont présents dans la paroi artérielle, en particulier dans l'intima. Sur des ostéoblastes en culture *in-vitro*, l'acide orthosilicique stimule la synthèse de collagène de type 1 et la différenciation des ostéoblastes (Reffitt *et al.,* 2003).

Des teneurs élevées de silicium ont été retrouvées dans des fibres alimentaires d'origine et de composition chimique différentes. Ces fibres ont montré un effet bénéfique sur la prévention de l'athérosclérose sur des modèles expérimentaux, sur la réduction des niveaux de cholestérol et lipides sanguins. En revanche d'autres fibres qui ne contenaient que des quantités négligeables de l'élément (différents types de celluloses purifiées par exemple) n'ont pas été efficaces. (Schwarz, 1977). Cependant, la nature chimique du silicium dans les différents types de fibres efficaces n'est pas connue. Il pourrait exister sous forme d'acide ortho-silicique, de polymères d'acide silicique, de silice colloïdale ou de produits dérivés de l'acide silicique liés organiquement (silanolates).

Des études préliminaires ont montré une relation inverse entre l'ingestion de silicium et le développement de l'athérosclérose chez l'homme (Schwarz *et al.,* 1979). Cet effet anti-athéromateux a été montré également chez le lapin (Loeper *et al.,* 1979). Ainsi, le manque de silicium peut être un facteur étiologique important dans l'athérosclérose, le taux de silicium décroissant avec l'âge et le développement de la pathologie. De plus, la présence de silicium dans le tissu aortique réduit la pression artérielle chez le rat spontanément hypertendu et supprime l'expression des gènes de l'angiogénèse dans la paroi aortique et des facteurs de croissance liés au remodelage vasculaire (Maehira *et al.,* 2011).

L'absorption de silicium chez l'homme est réalisée majoritairement sous forme d'acide orthosilicique Si(OH)₄ contenu dans les eaux de boisson ou formé après hydrolyse des aliments dans le tube digestif (Reffitt *et al.,* 1999). L'essentiel du silicium étant sous forme de silice colloïdale, il est peu absorbé par l'organisme. En effet, le silicium sous forme soluble se trouve essentiellement sous forme d'acide orthosilicique Si(OH)₄ dans les eaux de surface. Mais au-delà de 2mM et à pH neutre, l'acide orthosilicique polymérise en différentes formes de silice, silice colloïdale à silice solide (Reffitt *et al.,* 2003) peu ou pas soluble.

L'EFSA estime que l'apport journalier par l'alimentation est compris entre 20 et 50mg par jour soit 0,3 à 0,8mg/kg de poids corporel pour un homme de 60kg. (The EFSA Journal, 2004, 60, 1-11).

Il existe donc un besoin de disposer d'une composition comprenant une forme aisément métabolisable de silicium, et d'un procédé permettant la préparation d'une telle composition.

Ceci est maintenant l'objet de la présente invention. Les inventeurs ont en effet mis au point un procédé de production d'une composition enrichie en silicium sous une forme soluble dans l'eau et aisément métabolisable. De façon inattendue, une composition selon l'invention comprend du silicium sous une forme soluble dans l'eau, et ceci dans une proportion beaucoup plus importante que dans les compositions décrites dans l'état de l'art. Parmi les compositions de l'état de l'art, on peut citer en particulier la poudre de prêle, plante sauvage classiquement recommandée en herboristerie pour apporter du silicium sous forme « organique » et donc métabolisable par l'organisme. Une composition selon l'invention comprend une microalgue enrichie en silicium sous une forme soluble dans l'eau, une telle composition peut être produite par la mise en oeuvre d'un procédé selon l'invention.

Une microalgue est une algue microscopique, qui peut être procaryote ou eucaryote. Parmi les microalgues procaryotes, on peut citer les spirulines.

Les spirulines sont des microalgues très riches en protéines, de l'ordre de 70% de protéines par rapport à la matière sèche, qui sont un complément nutritionnel reconnu pour son efficacité et autorisées en alimentation humaine. Les spirulines sont souvent cultivées dans des bassins, ou éventuellement dans des réacteurs tubulaires, et ceci permet de contrôler les conditions de culture donc d'obtenir des résultats reproductibles quant à la composition de la matière végétale obtenue, par comparaison avec des cultures de végétaux réalisées en plein champ ou même sous serre.

Puyfoulhoux *et al*. (2001) décrit la production de spiruline enrichie en fer, obtenue par l'addition de FeCl3 dans le milieu de culture de la spiruline. Les résultats obtenus montrent que le fer extrait de spiruline est beaucoup mieux assimilé que le fer extrait de viande de boeuf. Cases *et al.* (1999 et 2002) décrivent la production de spiruline enrichie en sélénium, obtenue par l'addition de dioxyde de sélénium directement dans le milieu de culture de la spiruline. Ces documents ne décrivent pas une composition comprenant une microalgue enrichie en silicium soluble dans l'eau.

L'invention sera maintenant décrite de manière détaillée et illustrée d'exemples.

La présente invention a principalement pour objet une composition comprenant une microalgue enrichie en silicium sous une forme soluble dans l'eau, caractérisée en ce que la proportion de silicium total dans ladite composition est comprise entre 0,05% et 10%, de préférence entre 0,1% et 5% et de préférence entre 0,9% et 1,5% de la masse sèche de microalgue présente dans ladite composition.

Le terme « microalgue » désigne une algue microscopique photosynthétique, ladite microalgue peut être une microalgue eucaryote ou procaryote.

Le terme « microalgue enrichie en silicium » désigne une microalgue associée à du silicium présent en quantité supérieure par rapport à la quantité de silicium habituellement mesurée en association avec ladite microalgue lorsqu'elle est cultivée dans des conditions standard ou qu'elle est isolée de son milieu naturel.

Une composition selon l'invention comprend une microalgue enrichie en silicium dans laquelle le silicium peut être détecté dans le compartiment intracellulaire et/ou associé aux membranes et/ou associé à la surface de ladite microalgue.

Selon un aspect particulier, dans une composition selon l'invention, le silicium est détecté dans le compartiment intracellulaire.

Selon un autre aspect particulier, dans une composition selon l'invention, le silicium est détecté sous une forme associée aux membranes. Selon un autre aspect particulier, dans une composition selon l'invention, le silicium est détecté sous une forme associée à la surface de ladite micro algue.

Dans une composition selon l'invention, le silicium peut être détecté par tout moyen connu de l'homme du métier, notamment par l'observation microscopique, et de préférence par microscopie électronique couplée à une microsonde à rayons X, d'une composition selon l'invention. Lors de l'observation par microscopie électronique couplée à une microsonde à rayons X, des grains de silicium particulièrement denses peuvent être observés. Selon un autre aspect particulier, dans une composition selon l'invention, le silicium est détecté par RMN du ²⁹Si, ou RMN du solide ²⁹Si.

Le terme « silicium sous une forme soluble dans l'eau », ou éventuellement « silicium soluble dans l'eau », désigne du silicium présent et détectable dans un extrait aqueux d'une composition selon l'invention. Selon un aspect particulier de l'invention, ledit silicium sous une forme soluble dans l'eau est présent dans un extrait aqueux d'une composition selon l'invention comprenant une microalgue enrichie en silicium. La détection de la présence de silicium associé à une microalgue dans une composition selon l'invention peut être réalisée également par tout moyen chimique ou biochimique connu de l'homme du métier.

Le terme «silicium sous une forme soluble dans l'eau» ou « silicium sous une forme soluble » ou « silicium solubilisable » désigne du silicium disponible sous une forme réduite et pouvant être assimilée par l'organisme, contrairement au silicium minéral, qui est sous une forme oxydée, telle que la silice, qui peut précipiter et n'est pas assimilable par l'organisme. Le silicium minéral, sous une forme oxydée qui peut précipiter et n'est pas assimilable par l'organisme peut également être désigné par les termes « silicium polymérisé » et comprend notamment le composé désigné par « silice massive », qui est fortement polymérisée.

Dans une composition selon l'invention, le silicium présent sous une forme soluble dans l'eau peut se présenter sous une forme simple ou sous une forme complexée à une molécule organique, et peut être désigné dans ce cas par le terme « silicium organique ».

Selon un aspect particulier, dans une composition selon l'invention, le silicium présent sous une forme soluble dans l'eau est complexé à une molécule organique choisie parmi : les protéines, les sucres, les polysaccharides, les complexes entre protéines et lipides, protéines et sucres, lipides et sucres. Selon un aspect plus particulier, dans une composition selon l'invention, le silicium présent sous une forme soluble dans l'eau est détecté dans le compartiment intracellulaire, et complexé à une molécule organique choisie parmi : les protéines, les sucres, les polysaccharides, les complexes entre protéines et lipides, protéines et sucres, lipides et sucres.

Selon un aspect plus particulier, dans une composition selon l'invention, le silicium présent sous une forme soluble dans l'eau est complexé à une protéine choisie parmi : les phycobiliprotéines dont la phycocyanine, les protéines membranaires, les protéines de structure.

Selon un aspect encore plus particulier, dans une composition selon l'invention, le silicium présent sous une forme soluble dans l'eau est complexé à la phycocyanine.

Dans une composition selon l'invention, la proportion de silicium sous une forme soluble dans l'eau peut être déterminée par tout moyen connu de l'homme du métier, et notamment par l'extraction d'une composition selon l'invention par une solution aqueuse. De préférence, la proportion de silicium sous une forme soluble dans l'eau dans une composition selon l'invention peut être déterminée par extraction séquentielle de la composition par des solutions aqueuses d'agents actifs d'une composition selon l'invention. Si le silicium est présent sous une forme soluble dans l'eau, il sera solubilisé partiellement ou totalement au cours des extractions successives. Si le silicium est présent sous une forme polymérisée, il restera sous une forme insoluble malgré les extractions successives. L'extraction peut notamment être réalisée par : de l'eau, une solution enzymatique capable de dégrader les parois cellulaires végétales, une solution de détergent capable de solubiliser les fractions lipophiles, ou une solution enzymatique capable de digérer les protéines. La quantité de silicium présente dans un extrait aqueux, ou dans tout extrait d'une composition selon l'invention, peut être déterminée par toute méthode connue de l'homme de l'art, notamment par dosage gravimétrique.

Le terme « silicium total» désigne la quantité totale du silicium détectée dans une composition selon l'invention. Selon un aspect particulier de l'invention, le terme « silicium total» désigne la quantité totale du silicium détectée dans la masse sèche de microalgue comprise dans une composition selon l'invention. La quantité totale de silicium peut être déterminée par toute méthode connue de l'homme de l'art, notamment par dosage gravimétrique, par ICP-OES (Inductively Coupled Plasma-Optical Emission Spectrometry) ou par ICP-MS (Inductively Coupled Plasma-Mass Spectrum).

Le terme «masse sèche de microalgue » désigne la masse de microalgue présente dans la composition, déterminée après deshydratation de la microalgue, la déshydratation, ou le séchage, de ladite microalgue pouvant être réalisée par tout protocole choisi parmi les protocoles connus par l'homme de l'art. Le terme de « masse sèche de microalgue » peut être considéré comme équivalent au terme biomasse.

Une composition selon l'invention comprend une microalgue enrichie en silicium sous une forme soluble dans l'eau, la proportion de silicium total présent dans ladite composition étant comprise entre 0,05 % et 1, 2, 3, 4, 5, 6, 7, 8, 9 ou 10 % de la masse sèche de microalgue présente dans ladite composition. Selon un aspect particulier, dans une composition selon l'invention, la proportion de silicium total présent dans ladite composition est comprise entre 0,1% et 1, 2, 3, 4, 5, 6, 7, 8, 9 ou 10% de la masse sèche de microalgue présente dans ladite composition. Selon un aspect plus particulier, dans une composition selon l'invention, la proportion de silicium total présent dans ladite composition est comprise entre 0,5 et 5% de la masse sèche de microalgue présente dans ladite composition, de préférence entre 0,9% et 3%, plus préférentiellement entre 0,9% et 4% et encore plus préférentiellement entre 0,9% et 1,5%.

Selon un aspect particulier, une composition selon l'invention comprend une microalgue enrichie en silicium sous une forme soluble dans l'eau, caractérisée en ce que la proportion de silicium total dans ladite composition est comprise entre 0,05% et 10% de la masse sèche de microalgue présente dans ladite composition, et en ce que la proportion de silicium sous une forme soluble dans l'eau est comprise entre 5% et 90% du silicium total présent dans ladite composition.

Selon un aspect encore plus particulier, une composition selon l'invention comprend une microalgue enrichie en silicium sous une forme soluble dans l'eau, caractérisée en ce que la proportion de silicium total dans ladite composition est comprise entre 0,05% et 10% de la masse sèche de microalgue présente dans ladite composition, en ce que la proportion de silicium sous une forme soluble dans l'eau est comprise entre 5% et 90% du silicium total présent dans ladite composition et en ce que le silicium peut être détecté dans le compartiment cellulaire de ladite microalgue.

Selon un aspect encore plus particulier, une composition selon l'invention comprend une microalgue enrichie en silicium sous une forme soluble dans l'eau, caractérisée en ce que la proportion de silicium total dans ladite composition est comprise entre 0,9% et 1,5% de la masse sèche de microalgue présente dans ladite composition, en ce que la proportion de silicium sous une forme soluble dans l'eau est comprise entre 40% et 60% du silicium total présent dans ladite composition et en ce que le silicium peut être détecté dans le compartiment cellulaire de ladite microalgue.

Selon un aspect encore plus particulier, une composition selon l'invention comprend une microalgue enrichie en silicium sous une forme soluble dans l'eau, caractérisée en ce que la proportion de silicium total dans ladite composition est comprise entre 0,9% et 1,5% de la masse sèche de microalgue présente dans ladite composition, en ce que la proportion de silicium sous une forme soluble dans l'eau est comprise entre 41% et 58% du silicium total présent dans ladite composition, en ce que le silicium peut être détecté dans le compartiment cellulaire de ladite microalgue.

Selon un aspect encore plus particulier, une composition selon l'invention comprend une microalgue enrichie en silicium sous une forme soluble dans l'eau, caractérisée en ce que la proportion de silicium total dans ladite composition est comprise entre 0,9% et 1,5% de la masse sèche de microalgue présente dans ladite composition, en ce que la proportion de silicium sous une forme soluble dans l'eau est comprise entre 41% et 58% du silicium total présent dans ladite composition, et en ce que le silicium est détecté dans le compartiment cellulaire de ladite microalgue sous une forme complexée à la phycocyanine.

Selon un autre aspect particulier, une composition selon l'invention comprend une microalgue enrichie en silicium sous une forme soluble dans l'eau, ladite microalgue étant choisie parmi les microalgues eucaryotes. Selon un aspect plus particulier de l'invention, ladite microalgue eucaryote est choisie dans le groupe constitué par : les Chlorophycées (et de préférence *Dunaliella, Chlorella ou Parietochloris incisa*), les Chrysophycées, les Coccolithophyceae, les Diatomées, les Euglénophycées, les Rhodophycées et les Trebouxiophyceae.

Selon un autre aspect particulier, une composition selon l'invention comprend une microalgue ennrichie en silicium sous une forme soluble dans l'eau, ladite microalgue étant choisie parmi les microalgues procaryotes, aussi désignées par le terme « cyanobactéries » ou « algues bleues ». Selon un aspect plus particulier de l'invention, ladite microalgue procaryote est choisie dans le groupe constitué par les cyanophycées, parmi lesquelles la spiruline est particulièrement préférée.

Selon un aspect particulier, la présente invention a pour objet une composition comprenant une microalgue procaryote enrichie en silicium sous une forme soluble dans l'eau, ladite micro algue étant la spiruline.

Le terme « spiruline » désigne une algue filamenteuse bleue-verte appartenant au phyllum Cyanophyta, de la classe des Cyanophyceae, de l'ordre Nostocales, famille Oscillatoriaceae, et du genre Spirulina ou Arthrospira, et de préférence *Arthrospira platensis* ou *Arthrospira maxima.*

Selon un aspect plus particulier, la présente invention a pour objet une composition comprenant une microalgue procaryote enrichie en silicium sous une forme soluble dans l'eau, ladite microalgue étant *Arthrospira platensis.*

Selon un autre aspect plus particulier, la présente invention a pour objet une composition comprenant une microalgue procaryote enrichie en silicium sous une forme soluble dans l'eau, ladite microalgue étant *Arthrospira maxima.*

Selon un aspect plus particulier, l'invention a pour objet une composition comprenant une microalgue enrichie en silicium sous une forme soluble dans l'eau, caractérisée en ce que la proportion de silicium sous une forme soluble dans l'eau, déterminée par extraction successive dans de l'eau ou un solvant aqueux, est comprise entre 5% et 90%, de préférence entre 10% et 80%, entre 15% et 75%, entre 20% et 70%, entre 30% et 60%, et de préférence entre 40% et 60% du silicium total présent dans ladite composition.

Selon un aspect plus particulier, l'invention a pour objet une composition comprenant une microalgue enrichie en silicium sous une forme soluble dans l'eau et métabolisable, caractérisée en ce que la proportion de silicium sous une forme soluble dans l'eau et métabolisable est comprise entre 5% et 90%, de préférence entre 10% et 75%, entre 15% et 70%, entre 15% et 60%, entre 20% et 50%, entre 20% et 45%, entre 25% et 40%, entre 25% et 35%, et de préférence entre 25 et 30% du silicium total présent dans ladite composition.

Par « silicium sous une forme soluble dans l'eau et métabolisable », on entend du silicium biodisponible pour le corps après digestion, celui-ci peut être mesuré par un test de simulation de digestion artificielle, et notamment par simulation d'une digestion gastrique et simulation d'une digestion gastro-intestinale.

Selon un autre aspect, la présente invention a pour objet un procédé de production d'une composition comprenant une microalgue enrichie en silicium sous une forme soluble dans l'eau selon l'invention, ledit procédé comprenant les étapes suivantes :
a) la mise en culture de microalgues dans des conditions et un milieu de culture adaptés, jusqu'à l'obtention d'une culture de microalgues caractérisée par une biomasse sèche comprise entre 0,1 et 2 g/litre, de préférence entre 0,2 et 1 g/litre, de préférence entre 0,3 et 0,8 g/litre, et plus préférentiellement entre 0,4 et 0,6 g/litre de culture,
b) la préparation d'une solution aqueuse de silicium, le silicium étant ajouté sous la forme d'une solution, suivie de l'agitation de la solution aqueuse jusqu'à dissolution complète du silicium,
c) l'addition de la solution préparée en b) à la culture de microalgues obtenue en a),
d) la mise en culture des microalgues sous agitation pendant une durée d'au moins 12 heures,
e) la récolte d'au moins une partie de la culture de microalgues.

Dans un procédé selon l'invention, la culture de microalgues est réalisée dans des conditions adaptées à la culture des microalgues et connues de l'homme du métier. Plus particulièrement, dans un procédé selon l'invention, la culture des microalgues est réalisée dans des conditions permettant d'éviter les contaminations atmosphériques, notamment sous serre et dans un bassin permettant une pénétration optimale de la lumière, et donc l'activité photosynthétique, par exemple dans des bassins de 25 M³ et d'une faible profondeur, entre 15 et 25 cm, et de préférence d'environ 20 cm. En effet, le risque de récolter la spiruline croissant naturellement dans des lacs est de voir proliférer certaines cyanobactéries toxiques dans ces conditions non contrôlées.

Le mode de culture le plus répandu et le moins coûteux pour la spiruline est le bassin de type « raceway » dont l'agitation est assurée par une roue à aubes et avec une cloison centrale permettant de faire circuler la culture en circuit fermé. Ce mode de production permet d'isoler la culture dans des bassins de faible profondeur où les apports minéraux du milieu de culture artificiel sont contrôlés.

La culture est possible en cuve de 1 M³ avec le même milieu de culture et agitation par bullage à l'air comprimé ou par pompe. Un autre mode de culture est possible dans un photo réacteur tubulaire, essentiellement utilisé pour la culture de microalgues unicellulaires comme la chlorelle.

La culture de microalgues est généralement réalisée à un pH compris entre pH6 et pH12, de préférence à pH alcalin, plus préférentiellement à un pH compris entre pH9 et pH11, et plus préférentiellement à pH10. La culture peut être réalisée sous serre, en éclairage naturel et/ou artificiel, permettant de réaliser la photosynthèse, dans des conditions contrôlées de température, de pH et d'alimentation en dioxyde de carbone. L'agitation est réalisée par une ou des pompes, une roue à aubes ou un bullage par de l'air comprimé pour assurer la circulation de la culture et un brassage en profondeur. La température de culture est comprise entre 15 et 40°C avec un optimum à 33°C.

Dans un procédé selon l'invention, la durée de l'étape de culture de la microalgue dans des conditions et un milieu de culture adapté, jusqu'à obtention d'une culture caractérisée par une biomasse sèche comprise de préférence entre 0,4 et 0,6 g/litre, dépend de la concentration de l'ensemencement initial et des conditions de lumière et de température appliquées à la culture, elle est typiquement comprise entre 3 et 21 jours.

Dans l'étape b) de préparation d'une solution aqueuse de silicium d'un procédé selon l'invention, la dissolution de silicium est réalisée dans une solution aqueuse ou dans de l'eau, et de préférence de l'eau dé-ionisée ou purifiée c'est-à-dire ne contenant pas ou très peu de minéraux, et dans un volume restreint par rapport au volume final de la culture de microalgue, le volume d'eau dans lequel est dissous le silicium est typiquement compris entre 1/20^{ème} et 1/30^{ème} du volume final de culture. Dans un procédé selon l'invention, de l'eau dé-ionisée ou purifiée, c'est-à-dire ne contenant pas ou très peu de minéraux, est définie par sa minéralité, qui est de préférence comprise entre 5 et 15 µSiemens, de préférence voisine de 10 µSiemens et plus préférentiellement de 10 µSiemens.

Selon un aspect particulier d'un procédé selon l'invention, l'eau est filtrée et stérilisée par irradiation UV avant d'être utilisée pour la préparation de la solution et versée dans le bassin.

Dans un procédé selon l'invention, le silicium est ajouté à la solution aqueuse sous la forme d'une solution ou d'une poudre, avec agitation jusqu'à dissolution complète du silicium.

Selon un aspect particulier d'un procédé selon l'invention, le silicium dissous est ajouté à la solution aqueuse sous la forme d'une solution.

Le silicium sous forme soluble se trouve essentiellement sous forme d'acide orthosilicique Si(OH)₄ dans les eaux de surface. A une concentration supérieure à 2mM et à pH neutre, l'acide orthosilicique polymérise en différentes formes de silice, de colloïdale à solide (Reffitt *et al.,* 2003) peu ou pas soluble. Il est donc essentiel de le maintenir en solution afin qu'il reste absorbable par la microalgue, bien que le pH préférentiellement alcalin du milieu de culture d'une microalgue soit favorable à la solubilisation de l'acide orthosilicique.

Selon un aspect particulier d'un procédé selon l'invention, le silicium est ajouté à la solution aqueuse sous la forme d'une poudre, avec agitation jusqu'à dissolution complète du silicium. Selon un aspect encore plus particulier d'un procédé selon l'invention, le silicium est ajouté à la solution aqueuse sous la forme d'une poudre dont la granulométrie est inférieure à 30 microns, et de préférence est d'environ 10 microns.

Selon un aspect préféré d'un procédé selon l'invention, le silicium est ajouté sous une forme autre que la silice cristalline ou amorphe. Selon un autre aspect préféré d'un procédé selon l'invention, le silicium est ajouté sous une forme combinée, par exemple sous la forme de silicate.

Selon un aspect particulier d'un procédé selon l'invention, le silicium est ajouté sous la forme d'une composition choisie dans le groupe constitué par :
- du métasilicate de sodium ou du métasilicate de potassium, qui se dissout totalement dans l'eau et libère de l'acide orthosilicique en solution. Le métasilicate de sodium ou potassium se présente sous forme de poudre ou de solution aqueuse concentrée.
- un engrais ou toute composition contenant du silicium dont tout ou partie est sous forme soluble d'acide orthosilicique.
- un amendement silicique utilisé en agriculture.

Dans le cas d'une composition sous forme de poudre, celle-ci sera ajoutée sous la forme d'une poudre dont la taille des particules est inférieure à 30 microns, de préférence inférieure à 20 microns et plus préférentiellement inférieure à 10 microns. Ceci permet, lors de la récolte des spirulines par tamisage sur une toile filtrante de maille 30 µm, que les particules soient éliminées lors du tamisage, limitant ainsi la présence de silicium sous forme minérale dans la composition comprenant les spirulines.

Selon un aspect plus particulier d'un procédé selon l'invention, le silicium est ajouté sous la forme d'un engrais de type Agrosil broyé, caractérisé par des particules de taille inférieure à 10 microns.

Selon un autre aspect, la présente invention a pour objet un procédé de production d'une composition comprenant une microalgue enrichie en silicium sous une forme soluble dans l'eau selon l'invention, ledit procédé comprenant les étapes suivantes :
a) la mise en culture de microalgues dans des conditions et un milieu de culture adaptés, jusqu'à l'obtention d'une culture caractérisée par une biomasse sèche comprise entre 0,1 et 2 g/litre, de préférence entre 0,2 et 1 g/litre, de préférence entre 0,3 et 0,8 g/litre, et de préférence entre 0,4 et 0,6 g/litre de culture,
b) la préparation d'une solution aqueuse de silicium et de polyphénols, le silicium étant ajouté sous la forme d'une solution, ou d'une poudre, et les polyphénols étant ajoutés sous la forme d'une solution, suivie d'un mélange par agitation de la solution ainsi obtenue jusqu'à dissolution complète du silicium et des polyphénols,
c) l'addition de la solution préparée en b) à la culture de microalgues obtenue en a),
d) la mise en culture des microalgues sous agitation pendant une durée d'au moins 12 heures,
e) la récolte d'au moins une partie de la culture de microalgues.

Selon un aspect particulier d'un procédé selon l'invention, la préparation d'une solution aqueuse de silicium et de polyphénols est réalisée par l'addition de silicium à la solution aqueuse, suivie de l'addition de polyphénols.

Selon un autre aspect particulier d'un procédé selon l'invention, la préparation d'une solution aqueuse de silicium et de polyphénols est réalisée par l'addition de polyphénols à la solution aqueuse, suivie de l'addition de silicium.

Le terme « polyphénols » désigne un composé comprenant un ou plusieurs cycles benzéniques auxquels sont liés un ou plusieurs groupes hydroxyle, les groupes hydroxyle pouvant être libres ou engagés dans une autre fonction. Le terme « polyphénols » désigne donc un grand nombre de composés possibles, regroupant des molécules simples telles que les acides phénoliques à des composés hautement polymérisés comme les tanins, et sont caractérisés par un pouvoir anti-oxydant élevé.

Les polyphénols peuvent notamment être choisis parmi : les phénols simples tels que l'hydroquinone, les acides hydroxybenzoïques, les acides hydroxycinnamiques, les coumarines, les naphtoquinones, les stilbénoïdes, les flavonoïdes, les isoflavonoïdes, les anthocyanes, les lignanes, les lignines, les tanins condensés.

Les polyphénols sont présents dans diverses substances naturelles. C'est le cas notamment de l'anthocyanine dans les fruits rouges, des proanthocyanidines dans le chocolat et le vin, d'acides cafeoylquinique et feruloylquinique dans le café, de flavonoïdes dans les agrumes, de catéchines dans le thé vert et de quercétine dans les pommes.

Les composés phénoliques ont la propriété de se complexer partiellement avec les fonctions hydroxyles ionisées de l'acide orthosilicique en solution et ainsi de ralentir la polymérisation de l'acide orthosilicique.

Selon un aspect particulier d'un procédé selon l'invention, l'addition lors de l'étape c) de la solution de silicium et de polyphénols à la culture de microalgue obtenue à l'étape a) conduit à une culture de microalgue dans laquelle la concentration en silicium est comprise entre 0,1 et 20 g/litre de culture, de préférence entre 0,5 et 4 g/litre de culture, plus préférentiellement entre 1 et 3 g/litre de culture et plus préférentiellement de 2 g/litre de culture, et la concentration en polyphénols est comprise entre 1 et 300 mg/litre de culture, de préférence entre 5 et 60 mg/litre de culture.

Selon un autre aspect particulier d'un procédé selon l'invention, la solution de polyphénols ajoutée lors de l'étape b) est un extrait de polyphénols naturels.

Selon un aspect plus particulier d'un procédé selon l'invention, la solution de polyphénols est un extrait de polyphénols naturels choisi parmi des extraits végétaux. Un extrait végétal utilisable dans un procédé selon l'invention peut être choisi dans le groupe constitué par :
- un extrait de polyphénols d'olives, notamment un extrait de margines d'olive,
- un extrait de polyphénols de feuilles de thé,
- un extrait de polyphénol de raisins, notamment un extrait de marc et/ou de pépins de raisin, le raisin pouvant être blanc ou rouge,
- un extrait de polyphénol de gingembre ou de romarin, et
- un mélange de ces extraits.

Ces produits se présentent sous forme de poudre de solubilité variable dans l'eau ou sous forme aqueuse concentrée. Les polyphénols d'olive ou de raisin présentent l'avantage d'être produits localement en grandes quantités et constituent des sous-produits peu coûteux des filières viticoles et oléicoles.

Selon un autre aspect particulier d'un procédé selon l'invention, la solution de polyphénols comprend au moins un polyphénol d'origine synthétique. Une préparation d'acide orthosilicique stabilisée par de la choline a été autorisée comme complément alimentaire par l'EFSA (The EFSA Journal, 2009, 948, 1-23). La choline pourrait donc être utilisée comme composé permettant de maintenir l'acide orthosilicique en solution. Cependant, la choline est un produit assez coûteux et il faudrait en utiliser des quantités importantes par bassin de 25M³ en substitution des polyphénols de raisin, d'olive, de thé ou d'autres plantes.

Selon un aspect particulier d'un procédé selon l'invention, les microalgues sont récoltées par tamisage, centrifugation ou floculation, et de préférence par tamisage. Selon un aspect préféré, la microalgue est récoltée par tamisage sur une toile filtrante de maille comprise entre 20 et 40 microns, et de préférence de 30 microns.

Dans un procédé selon l'invention, la récolte d'une fraction de la culture de microalgue commence après un temps d'incubation de la microalgue avec le silicium, et éventuellement les polyphénols compris entre 12H et 3 jours ; durant cette période, la biomasse de spiruline augmente d'au moins 20%. Selon un aspect particulier d'un procédé selon l'invention, la récolte de fractions successives de la culture de microalgue est ensuite réalisée tous les jours, selon l'état de la culture et ceci pendant une durée de 2 à 8 semaines, préférentiellement environ 3 semaines. Selon un autre aspect particulier d'un procédé selon l'invention, une nouvelle solution aqueuse de silicium, comprenant préférentiellement des polyphénols, est ajoutée environ 3 semaines après la récolte, dans le bassin de culture, pour un nouveau cycle d'enrichissement.

La quantité de microalgue récoltée chaque jour est déterminée pour ne pas mettre en péril toute la culture. Cette quantité varie de 5% à 15% de la quantité totale de microalgue dans la culture. Après chaque récolte partielle, une quantité calculée d'éléments minéraux du milieu de Zarouk, ou de tout autre milieu de culture utilisable pour ce procédé, est ajoutée dans le bassin afin de compenser les éléments minéraux consommés par les microalgues récoltées. Il est aussi possible de récolter la totalité des microalgues du bassin au bout de 8 jours de culture.

Selon un aspect particulier d'un procédé selon l'invention, la microalgue récoltée est filtrée puis séchée pour obtenir une composition de microalgue déshydratée ou partiellement déshydratée. De préférence, le séchage est effectué à une température inférieure à 60°C, pour éviter de dénaturer les principes actifs contenus dans les compositions de microalgue. Selon un aspect encore plus particulier d'un procédé selon l'invention, la poudre séchée de micro algue est broyée.

Selon un aspect particulier d'un procédé selon l'invention, après l'addition de la solution aqueuse de silicium préparée lors de l'étape b), la concentration de silicium dans le milieu de culture lors de l'étape c) est comprise entre 0,1 et 20 g/litre de culture, de préférence entre 0,5 et 4 g/litre, de préférence entre 1 et 3 g/litre, et plus préférentiellement de 2 g/litre. L'apport des 2g/litre de silicium peut être réalisé en une seule fois ou de façon séquentielle, par exemple en 2 jours consécutifs en apportant 1g/litre de silicium le premier jour et 1g/litre de silicium le deuxième jour.

Selon un autre aspect particulier d'un procédé selon l'invention, la concentration en polyphénols dans la culture lors de l'étape c) est comprise entre 5 et 60 mg/litre, de préférence entre 10 et 20 mg/litre et plus préférentiellement de 12 mg/litre de culture.

L'apport des 2g/litre de silicium et de polyphénols peut être réalisé en une seule fois ou de façon séquentielle, par exemple en 2 jours consécutifs en apportant 1g/litre de silicium et de polyphénols le premier jour et 1g/litre de silicium et de polyphénols le deuxième jour.

Selon un autre aspect particulier d'un procédé selon l'invention, le milieu de culture est choisi dans le groupe constitué par : un milieu de culture de cyanobactéries, un milieu organique constitué d'eau additionnée d'une solution concentrée (de type Antys8) et de NaHCO3, un milieu organique constitué d'eau additionnée d'une solution d'engrais commerciale concentrée (de type Algoflash) et de NaHCO3.

L'invention a également pour objet une composition comprenant une microalgue enrichie en silicium sous une forme soluble dans l'eau, susceptible d'être obtenue par la mise en oeuvre d'un procédé selon l'invention. Une telle composition est notamment le produit de récolte des microalgues cultivées, mais aussi toute composition issue de la transformation de ce produit de récolte en un produit dérivé. Une composition selon l'invention comprend essentiellement une microalgue enrichie en silicium sous une forme soluble dans l'eau comprenant également un ou plusieurs produit choisi parmi un excipient, un additif artificiel, un additif naturel, un ou plusieurs principes actifs, tel qu'une ou plusieurs vitamines, un adjuvant, tel qu'un agent d'enrobage.

Une composition selon l'invention peut être sous une forme solide ou liquide, sous la forme d'une poudre, de comprimés simples ou dragéifiés, de gélules, de granulés, de paillettes ou de sirop.

L'invention a également pour objet une composition comprenant une microalgue enrichie en silicium sous une forme soluble dans l'eau selon l'invention, ou susceptible d'être obtenue par un procédé selon l'invention, destinée à la préparation d'un médicament. L'invention a également pour objet une composition pharmaceutique comprenant une composition selon l'invention comprenant une microalgue enrichie en silicium sous une forme soluble dans l'eau et un excipient pharmaceutiquement acceptable.

L'invention a plus particulièrement pour objet une composition comprenant de la spiruline enrichie en silicium sous une forme soluble dans l'eau selon l'invention, ou susceptible d'être obtenue par un procédé selon l'invention, destinée à la préparation d'un médicament. L'invention a également pour objet une composition pharmaceutique comprenant de la spiruline enrichie en silicium sous une forme soluble dans l'eau selon l'invention et un excipient pharmaceutiquement acceptable.

L'invention a également pour objet une composition cosmétique comprenant une composition selon l'invention comprenant une microalgue enrichie en silicium sous une forme soluble dans l'eau, selon l'invention, et un excipient cosmétiquement acceptable.

L'invention a également pour objet une composition cosmétique comprenant une composition selon l'invention comprenant de la spiruline enrichie en silicium sous une forme soluble dans l'eau, selon l'invention, et un excipient cosmétiquement acceptable.

L'invention a également pour objet une composition alimentaire, ou un complément alimentaire, comprenant une composition comprenant une microalgue enrichie en silicium sous une forme soluble dans l'eau selon l'invention.

L'invention a également pour objet une composition alimentaire, ou un complément alimentaire, comprenant une composition comprenant de la spiruline enrichie en silicium sous une forme soluble dans l'eau selon l'invention.

D'autres caractéristiques et avantages de l'invention apparaissent dans les exemples et figures suivants.

### LEGENDES DES FIGURES

Figure 1 : Photo à la loupe binoculaire (grossissement: x40), lot 1 de spiruline enrichie en silicium, préparée selon l'exemple 1. On n'observe pas de grains de silice.
Figure 2 : Photo à la loupe binoculaire (grossissement: x40), lot 3 de spiruline enrichie en silicium, préparée en l'absence d'extraits de polyphénols. On observe une présence importante de grains de silice insoluble.
Figure 3 : Observation en microscopie électronique à balayage de spiruline enrichie en silicium, préparée selon l'exemple 1.
Figure 4 : Spectre RMN du solide ²⁹Si de la poudre de métasilicate de sodium.
Figure 5 : Spectre RMN du solide ²⁹Si de la phycocyanine extraite de spiruline enrichie en silicium, préparée selon l'exemple 1.
Figure 6 : Spectre RMN du solide ²⁹Si de la phycocyanine extraite de spiruline cultivée dans un milieu de culture contenant du silicium qui n'a pas été apporté par le procédé selon l'invention.

### EXEMPLES

### Exemple 1 : Protocole d'enrichissement de spiruline en silicium

La souche de spiruline utilisée est *Arthrospira platensis.* La souche *Arthrospira maxima* peut également être utilisée dans un procédé selon l'invention.

La culture de spiruline avant enrichissement en silicium est réalisée dans le milieu Zarouk de référence pour les cyanobactéries dont la composition est la suivante :
16.8 g/L NaHCO3 ; 0.5 g/L K2HP04 ; 2.5 g/L NaNO3 ; 1.0 g/L K2S04 ; 1.0 g/L NaCl; 0.2 g/L MgSO4-7H2O ; 0.04 g/L CaC12 ; 0.01 g/L FeSO4-7 H2O ; 0.08 g/L EDTA 2.86x10⁻³ g/L H3B03 ; 1.81x10⁻³ g/L MnCl2-4 H2O ; 0.22x10⁻³ g/L ZnSO4-7 H2O 7.9x10⁻⁵ g/L CuSO4-5 H2O ; 1.5x10⁻⁵ g/L MoO3 ; 2.1x10⁻⁵ g/L Na2MoO₄

D'autres milieux de culture adaptés aux cyanophycées peuvent aussi être utilisés dans un procédé selon l'invention. Le pH du milieu Zarouk est naturellement d'environ pH10. Les éléments sont dissous dans l'eau provenant d'un forage. Cette eau est filtrée et stérilisée par irradiation UV avant d'être versée dans le bassin.

La culture est réalisée sous serre en éclairage naturel, dans un bassin de type « raceway » d'un volume de 25 M³ comprenant une cloison centrale permettant de faire circuler la culture en circuit fermé. La profondeur de la culture est de 15 à 20 cm. L'agitation de la culture est apportée par des pompes, ou roues à aubes, ou bullage par de l'air comprimé, afin d'assurer la circulation de la culture et un brassage en profondeur. La température optimum de culture de 33°C, elle peut être comprise entre 15 et 40°C.

Une solution initiale de métasilicate de sodium et de polyphénols est préparée dans un volume de 1 M³ avant d'être versée dans les 25 M³ du bassin. La solution peut également être préparée à partir de la même concentration de métasilicate de potassium.

Pour un bassin de 25 M³, 50 kg de métasilicate de sodium sont dissous dans un volume de l'ordre de 1 M³ d'eau dé-ionisée. Après dissolution complète du métasilicate de sodium, 300 g de polyphénols de thé sont ajoutés à la solution et celle-ci est agitée pendant 30 minutes. Les polyphénols de thé sont ajoutés sous la forme d'un extrait de feuilles de thé vert (Camellia sinensis (L.) Kuntze), sous la forme d'une poudre brune dispersible dans l'eau, contenant 98% de polyphénols totaux, composés de 75% de catéchines dont 40% de EGCG (Epigallocatechine gallate) et 0,5% de caféine (Naturex, France, Ref. EA140374). Le métasilicate va réagir avec les polyphénols et se stabiliser. La solution est ensuite doucement versée dans le bassin de 25 M³ contenant la culture de spiruline déjà présente à une concentration en biomasse sèche de 0,5g/litre environ.

La concentration de métasilicate de sodium dans la solution initiale est de 50 g/litre afin d'obtenir une concentration finale en métasilicate de sodium de 2g/litre de culture dans les 25 M³ du bassin. La concentration de polyphénols est de 0,3 g/litre dans la solution initiale, elle atteint 0,012g/litre dans le bassin de culture. L'apport des 2g/litre de métasilicate de sodium associé aux polyphénols pourra être réalisé en une seule fois ou en 2 jours consécutifs en apportant 1g/litre de métasilicate de sodium associé aux polyphénols le premier jour et 1g/litre de métasilicate de sodium associé aux polyphénols le deuxième jour.

Après culture de la spiruline en présence de silicium et de polyphénols pendant une durée comprise entre 12 heures et 3 jours, la culture est récoltée en partie ou en totalité. La récolte d'une partie de la culture de spiruline est effectuée par tamisage, centrifugation ou floculation, après un temps d'incubation compris entre 12H et 3 jours, puis tous les jours selon l'état de la culture et ceci pendant une durée de 2 à 8 semaines, préférentiellement pendant 3 semaines. La récolte de la spiruline par tamisage permet l'élimination du silicium présent dans le milieu de culture et non intégré par les spirulines.

La quantité de spiruline récoltée chaque jour est déterminée afin de ne pas mettre en péril l'ensemble de la culture. Cette quantité varie de 5% à 15% de la quantité totale de spiruline présente. Cependant, il est possible de récolter la totalité des spirulines du bassin au bout de 8 jours et recommencer une nouvelle culture.

Après chaque récolte partielle, une quantité calculée d'éléments minéraux du milieu de Zarouk est ajoutée dans le bassin afin de compenser les éléments minéraux consommés par les spirulines récoltées.

Il est également possible, 3 semaines après une récolte de spiruline, d'ajouter dans le bassin de culture une nouvelle solution de la solution concentrée de métasilicate et de polyphénols, préparée selon le protocole indiqué précédemment, pour un nouveau cycle d'enrichissement.

La culture de la spiruline est également possible en cuve de 1 M³ avec le même milieu de culture Zarouk et sous agitation par bullage à l'air comprimé ou par pompe. La culture de la spiruline est également possible dans un photobioréacteur tubulaire, en utilisant également le milieu de culture Zarouk. Les volumes de culture dans ces protocoles seront adaptés pour une culture en cuve de 1M³ ou en photoréacteur tubulaire, cependant les concentrations finales en métasilicate et polyphénols resteront inchangées.

La spiruline récoltée peut ensuite être deshydratée ou préparée selon un protocole adapté à son utilisation.

### Exemple 2 : Détermination de la proportion de silicium dans la spiruline

La proportion de silicium dans les spirulines récoltées est déterminée après différentes durées de culture de spiruline en présence de silicium, par dosage gravimétrique. Brièvement, de la poudre de spiruline enrichie en silicium préparée selon l'exemple 1 est pesée dans une coupelle en platine puis chauffée à 700°C, les cendres qui en résultent et contiennent les minéraux sont traitées par de l'acide fluorhydrique. Les composés fluorés volatils s'évaporent, à l'exception du silicium, présent sous forme de silice SiO2. La coupelle est ensuite pesée afin de déterminer la masse de SiO2 présente. La masse de silicium est estimée par calcul, sur la base d'une proportion de 46,74 % de silicium dans la silice.

Dans le premier essai, les résultats sont les suivants :

**Tableau 1**

| Durée de la culture | Proportion de silicium par rapport à la matière sèche de spiruline récoltée |
|---|---|
| T=0 | 0% |
| T=7 jours | 1,95% |
| T=11 jours | 2,07% |
| T=12 jours | 1,90% |
| T=13 jours | 1,16% |
| T=14 jours | 1,04% |
| T=18 jours | 0,22% |
| T=20 jours | 0,17% |
| T=21 jours | 0,07% |
| T=25 jours | 0% |

Dans un deuxième essai, les résultats sont les suivants :

**Tableau 2**

| Durée de la culture | Proportion de silicium par rapport à la matière sèche de spiruline récoltée |
|---|---|
| T=0 | 0% |
| T=4 jours | 0% |
| T=5 jours | 0% |
| T=8 jours | 1,58% |
| T=9 jours | 1,93% |

### Exemple 3 : Caractérisation de la forme de silicium présente dans les spirulines

Lorsqu'il est sous forme de silice (SiO₂) le silicium peut être polymérisé et n'est pratiquement pas absorbable par l'organisme. Pour être absorbable, ou métabolisable, lorsqu'il est ingéré sous forme de complément alimentaire, le silicium doit nécessairement être sous une forme soluble dans une solution aqueuse.

Pour déterminer sous quelle forme chimique est présent le silicium dans la spiruline, des extractions séquentielles de poudre de spiruline riche en silicium par des solutions aqueuse d'agents de plus en plus actifs ont été réalisées. A chaque étape, la suspension de poudre dans la solution d'agents actifs est centrifugée, le surnageant conservé pour réaliser un dosage de silicium global et le culot de centrifugation récupéré et traité par la solution d'agent suivante. Le protocole est répété pour toute la série d'agents d'extraction et le culot final résiduel est récupéré pour réaliser un dosage du silicium résiduel insoluble. Si le silicium est présent sous forme métabolisée disponible, il sera tout ou partie solubilisé au cours des extractions successives. S'il existe sous forme polymérisée, il restera insoluble.

Pour comparaison, le même protocole a été appliqué à de la poudre de prêle (*Equisetum arvense*), plante sauvage naturellement riche en silicium et qui est classiquement recommandée en herboristerie dans le but d'apporter du silicium sous une forme métabolisable, parfois dite « organique ».

Les différentes étapes d'extraction successives sont les suivantes :
- Extraction à l'eau
- Extraction par une solution de Driselease, mélange d'enzymes (cellulase, hemi-cellulase, pectinase) capables de dégrader les parois cellulaires végétales.
- Extraction par une solution de SDS (sodium dodecyl sulfate), agent détergent capable de solubiliser les fractions lipophiles
- Extraction par une solution de protéase

Le protocole d'extractions séquentielles successives est le suivant :
0,2 g de poudre de spiruline enrichie en silicium sont extraits successivement avec :
- 5 ml d'eau chaude (85-90°C), agitation pendant 1H pour les formes solubles dans l'eau de silicium. Après centrifugation, le surnageant est récupéré pour le dosage de silicium et le culot repris par la solution suivante.
- 5ml de Driselase à 4% dans un tampon Tris-HCl 30mM pH7, agitation pendant 1H à 25°C pour extraire les formes de silicium associées aux parois cellulaires. Après centrifugation, le surnageant est récupéré pour le dosage de silicium et le culot repris par la solution suivante.
- 5ml de tampon Tris HCl 30mM pH7 contenant 4% de SDS, agitation pendant 1H à 25°C pour extraire les formes de silicium associées aux protéines insolubles. Après centrifugation, le surnageant est récupéré pour le dosage de silicium et le culot repris par la solution suivante.
- 5ml de tampon phosphate pH7,5 contenant un mélange de 10mg de lipase et 20mg de Pronase, incubation pendant 16H à 37°C pour libérer et extraire les protéines résiduelles contenant du silicium. Après centrifugation, le surnageant est récupéré pour le dosage de silicium et le culot redissous complètement dans 2ml d'une solution aqueuse à 25% de tetramethyl ammonium hydroxyde (TMAH) pour déterminer le silicium résiduel.

Le pourcentage de silicium contenu dans les poudres est mesuré avant le protocole d'extraction par dosage gravimétrique ou par dosage par ICP-OES (Inductively Coupled Plasma-Optical Emission Spectrometry) avec étalonnage externe après minéralisation en milieu acide (HNO3/HF) sous champ micro-ondes et dans chacune des fractions par dosage par ICP-OES avec étalonnage externe après minéralisation en milieu acide (HNO3/HF) sous champ micro-ondes

Les résultats sont les suivants :

**Tableau 3**

| | Spiruline silicium Lot 1 | Spiruline silicium Lot 2 | Poudre de prêle |
|---|---|---|---|
| Teneur en silicium de la poudre avant extraction | 1,07% | 0,91% | 7,7% |
| % cumulé de silicium extrait à l'issue des 5 étapes | 0,62% | 0,37% | 0,6% |
| % de silicium résiduel dans le culot | 0,45% | 0,54% | 7,1% |
| % de silicium sous une forme soluble dans l'eau, dans la poudre avant extraction | **58%** | **41%** | **7,8%** |

Nous constatons que bien que la teneur en silicium de la poudre de prêle soit élevée (7,7%) seulement 7,8% du silicium contenu dans les extraits de prêle est soluble, alors que cette proportion atteint 58% et 41% dans deux lots différents de spiruline enrichie en silicium. Le silicium métabolisé dans la spiruline est bien présent sous une forme soluble dans l'eau, ou biodisponible, contrairement à la poudre de prêle.

### Exemple 4 : Caractérisation de la digestibilité du silicium de la spiruline enrichie en silicium directement obtenue par le procédé selon l'invention

Une simulation de digestion artificielle, comprenant la simulation de la digestion gastrique puis intestinale, a été réalisée afin de comparer différents lots de poudre de spiruline enrichie en silicium, préparée suivant un procédé selon l'invention, de la poudre de spiruline disponible sur le marché et de la poudre de prêle. Le silicium présent dans le liquide obtenu après la digestion a été dosé, il constitue le silicium libéré et donc biodisponible pour le corps. Ce test permet de recréer *in-vitro* une simulation des conditions de digestion chez l'homme. Comparé au test précédent, celui-ci est beaucoup plus proche des conditions réelles de la digestion humaine. L'objectif est de mesurer le pourcentage de silicium qui sera libéré par la poudre de l'échantillon lors de la digestion et donc disponible pour être absorbé par l'intestin. La comparaison avec les pourcentages de silicium obtenus avec les autres échantillons donnera une indication importante sur la biodisponibilité du silicium contenu dans la poudre de spiruline enrichie. Le pourcentage initial de silicium dans chacun des produits est exprimé en pourcentage de la masse sèche du produit.

Le test est réalisé en deux étapes :
1. Simulation des conditions gastriques : l'échantillon est mis en suspension dans une solution aqueuse à pH acide et contenant des enzymes gastriques (pepsine). La suspension est agitée pendant 2H à 37°C. A l'issue de l'incubation, la suspension est centrifugée, le culot est récupéré pour l'étape suivante et le surnageant conservé pour dosage du silicium solubilisé dans le milieu aqueux.
2. Simulation des conditions gastro-intestinales : le culot récupéré lors de l'étape précédente est mis en suspension dans une solution aqueuse à pH basique et contenant des enzymes intestinales (lipases, protéase). La suspension est agitée pendant 2H à 37°C. A l'issue de l'incubation, la suspension est centrifugée et le surnageant conservé pour dosage du silicium solubilisé dans le milieu aqueux.

Le dosage du silicium soluble dans chacune des fractions est réalisé par ICP-OES (Inductively Coupled Plasma-Optical Emission Spectrometry) avec étalonnage externe après minéralisation en milieu acide (HNO3/HF) sous champ micro-ondes. Les échantillons testés sont :
- Les deux lots 1 et 2 de spiruline enrichie en silicium tels que décrits dans l'exemple 3.
- Un lot de spiruline enrichie en silicium sans avoir utilisé de polyphénols lors du protocole d'enrichissement, tel qu'obtenu dans l'exemple 1. A l'observation par loupe binoculaire, la poudre obtenue présente entre les filaments de nombreux petits grains de silicium polymérisé en silice.
- De la poudre de prêle (*Equisetum arvense*) contenant 7,7% de silicium. Elle est recommandée en herboristerie pour apporter du silicium sous forme « organique ».
- Un extrait de poudre de prêle. Il s'agit d'un extrait aqueux commercialisé, obtenu par extraction à l'eau de poudre de prêle. Selon la notice, il est recommandé pour apporter du « silicium d'origine naturelle ». Cet extrait liquide a été séché et le pourcentage de silicium a été dosé sur la poudre obtenue. Le résultat est de 4,17% de silicium.
- Un produit commercial contenant de la spiruline et du silicium. Un dosage de silicium a été réalisé selon le protocole utilisé pour les autres poudres et le résultat est de 0,2%. Selon la notice, la biodisponibilité annoncée du silicium est supérieure à 80%.
- *Odontella aurita* : il s'agit d'une diatomée, microalgue alimentaire contenant naturellement du silicium. Le dosage de silicium mesuré est de 7%. Comme la poudre de prêle, cette microalgue est classiquement recommandée en herboristerie pour apporter du silicium sous forme « organique ».

Les résultats sont les suivants :

**Tableau 4**

| | Spiruline enrichie en Si (Lot 1) | Spiruline enrichie en Si (Lot 2) | Spiruline enrichie en Si (Lot 3) | Poudre de prêle | Extrait sec de poudre de prêle | Produit commercial | Odontella aurita |
|---|---|---|---|---|---|---|---|
| Teneur initiale en Si | 1,07% | 0,91% | 7,25% | 7,7% | 4,17% | 0,21% | 7% |
| % de Si solubilisé dans l'extrait gastrique | 10,8% | 9,7% | 1,88% | 0,3% | 0,002% | 0% | 0% |
| % de Si solubilisé dans l'extrait gastro-intestinal | 16,5% | 13,2% | 3,54% | 0,7% | 0% | 0% | 0% |
| % total de Si solubilisé (extrait gastrique + gastro-intestinal) | **27.3%** | **22.9%** | **5.42%** | **1%** | **0.002%** | **0%** | **0%** |

Il apparaît très clairement que l'extrait de poudre de prêle, le composé commercial et *Odontella aurita* ne libèrent aucune forme soluble de silicium. L'extrait de prêle est cependant annoncé comme contenant du silicium sous forme soluble. Seule la poudre de prêle libère 1% de silicium soluble. Le produit commercial est annoncé contenir 1,7% de silicium, alors que nous avons mesuré un taux de 0,21 %.

Seuls les deux lots 1 et 2 de spiruline enrichie en silicium selon le procédé de l'invention libèrent 23 à 27% de leur silicium lors de cette digestion artificielle. Il est important de noter que, dans le lot 3 de spiruline enrichie en silicium mais sans stabiliser le métasilicate par les polyphénols de thé lors du protocole d'enrichissement, le pourcentage de silicium solubilisé est plus faible : de 5,42% contre 27,3% et 22,9% pour les lots de spiruline enrichie en silicium selon le protocole décrit précédemment. Ces résultats confirment l'intérêt de la présence des polyphénols comme composants stabilisateurs lors de la phase de solubilisation du métasilicate afin de prévenir sa polymérisation sous forme de grains de silice insoluble.

Cependant, quel que soit le protocole utilisé dans une méthode selon l'invention, le pourcentage de silicium solubilisé à partir d'un lot de spiruline enrichie en silicium selon un procédé de l'invention est supérieur au pourcentage de silicium solubilisé à partir d'extraits de prêle ou de compositions de spiruline disponibles sur le marché.

Les résultats de cette étude confirment ceux obtenus lors de l'étude d'extractions successives des formes solubles de silicium de l'exemple précédent. L'ordre du classement final des échantillons est conservé dans les résultats des deux méthodes d'analyse.

### Exemple 5 : Observation microscopique de la spiruline

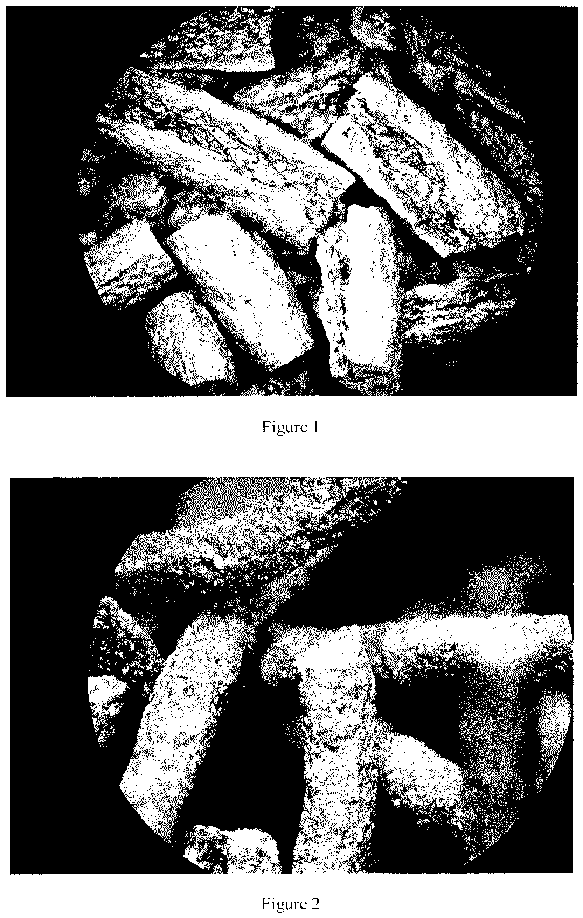

Un premier lot de spiruline enrichie en silicium est préparé selon le protocole décrit dans l'exemple 1 (Lot 1), un autre lot est préparé suivant un protocole identique à celui qui est décrit dans l'exemple 1 à l'exception de l'addition de polyphénols de thé (Lot 3). Chacun des lots est observé à la loupe binoculaire. On n'observe pas la présence de grains de silice sous une forme insoluble dans le lot 1, alors qu'une présence importante de grains de silice sous une forme insoluble est observée dans le lot 3 (Figures 1 et 2).

### Exemple 6 : Observation en microscopie à balayage et analyse élémentaire par microsonde en rayons X.

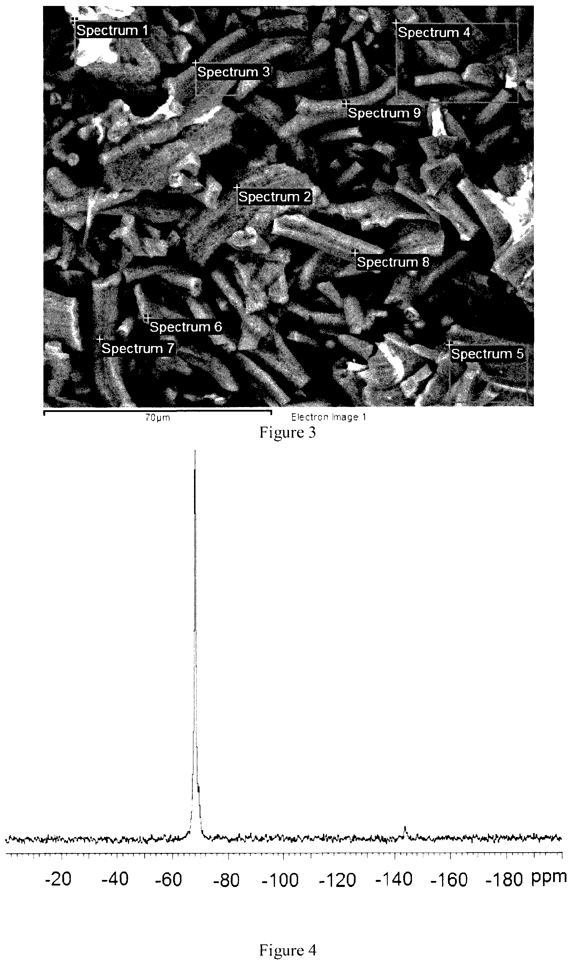

La Figure 3 montre la photographie de spirulines sèches enrichies en silicium selon le procédé. Les rectangles colorés ou les croix indiquent les surfaces ou les points analysées par la microsonde en rayons X. Le tableau ci-dessous indique les valeurs en pourcentage des éléments dosés par la microsonde, le silicium est indiqué en rouge.

**Tableau 5**

| Spectre | Stat. | C | N | O | Na | Mg | Si | P | S | K | Ca | Total |
|---|---|---|---|---|---|---|---|---|---|---|---|---|
| Spectre 1 | Non | 49.62 | 8.53 | 20.89 | 0.25 | 1.64 | 11.07 | 1.11 | 0.72 | 5.85 | 0.31 | 100.00 |
| Spectre 2 | Oui | 58.07 | 12.29 | 26.28 | 0.47 | 0.35 | 0.18 | 0.85 | 0.53 | 0.88 | 0.08 | 100.00 |
| Spectre 3 | Yes | 51.32 | 13.59 | 32.67 | 0.34 | 0.30 | 0.19 | 0.52 | 0.42 | 0.61 | 0.06 | 100.00 |
| Spectre 4 | Yes | 53.79 | 10.22 | 30.85 | 0.46 | 0.43 | 0.79 | 1.24 | 0.75 | 1.42 | 0.04 | 100.00 |
| Spectre 5 | Yes | 53.22 | 10.10 | 30.84 | 0.33 | 0.48 | 0.58 | 1.35 | 0.87 | 1.89 | 0.34 | 100.00 |
| Spectre 6 | Yes | 54.04 | 13.65 | 28.64 | 0.38 | 0.41 | 0.15 | 1.15 | 0.62 | 0.94 | 0.03 | 100.00 |
| Spectre 7 | Yes | 58.13 | 9.58 | 27.25 | 0.56 | 0.43 | 0.37 | 1.15 | 0.99 | 1.50 | 0.05 | 100.00 |
| Spectre 8 | Yes | 58.91 | 9.18 | 25.32 | 0.62 | 0.61 | 0.44 | 1.69 | 1.32 | 1.76 | 0.15 | 100.00 |
| Spectre 9 | Yes | 58.38 | 10.47 | 25.85 | 0.61 | 0.50 | 0.22 | 2.22 | 0.54 | 1.12 | 0.10 | 100.00 |
| | | | | | | | | | | | | |
| Moyenne | | 55.73 | 11.13 | 28.46 | 0.47 | 0.44 | 0.36 | 1.27 | 0.76 | 1.26 | 0.11 | 100.00 |
| Déviation std | | 2.95 | 1.78 | 2.73 | 0.12 | 0.09 | 0.23 | 0.51 | 0.30 | 0.45 | 0.10 | |
| Max. | | 58.91 | 13.65 | 32.67 | 0.62 | 0.61 | 0.79 | 2.22 | 1.32 | 1.89 | 0.34 | |
| Min. | | 51.32 | 9.18 | 25.32 | 0.33 | 0.30 | 0.15 | 0.52 | 0.42 | 0.61 | 0.03 | |

Tous les résultats sont exprimés en pourcentage de poids.

Interprétation : Sur la photographie, les filaments de spiruline apparaissent clairs, sans présence de silice polymérisée, à l'exception d'un petit grain analysé dans le spectre N°1, qui ne représente cependant qu'une faible proportion comparé à l'ensemble de la masse de spiruline analysée._Les autres spectres (N°2 à 7) correspondent aux mesures dans les zones vierges de silice polymérisée et le silicium y est détecté.
Le silicium a donc bien été pratiquement totalement métabolisé à l'intérieur de la spiruline.

### Exemple 7 : Analyse par RMN du solide ²⁹Si de poudre de metasilicate de sodium, de phycocyanine extraite de spiruline enrichie en silicium selon le procédé et de phycocyanine extraite de spiruline enrichie en silicium sans le procédé.

La RMN du solide ²⁹Si permet d'obtenir des informations sur la nature des liaisons chimiques entre les atomes de silicium, directement à partir d'un échantillon de poudre.
Ces informations permettent de mieux caractériser le silicium présent dans la spiruline enrichie en silicium selon le procédé. Cette technique est limitée par le fait que_les signaux RMN du ²⁹Si présentent une faible intensité comparée aux signaux RMN du carbone ou de l'hydrogène. Pour obtenir un spectre exploitable, le temps d'analyse est très long.

### 1. Analyse par RMN du solide ²⁹Si de poudre de métasilicate de sodium

Le métasilicate de sodium (Na₂SiO₃) constitue une des sources possible de silicium apportée durant la culture de la spiruline, dans un procédé selon l'invention. L'analyse par RMN du solide ²⁹Si permet de déterminer si les signaux obtenus avec ce composé sont différents de ceux observés avec la spiruline enrichie en silicium et ainsi démontrer que le silicium présent dans la spiruline enrichie selon le procédé n'est pas un simple résidu de métasilicate de sodium

Le spectre obtenu est représenté dans la figure 4. Le signal obtenu se situe à -68ppm.

### 2. Analyse RMN du solide ²⁹Si de la phycocyanine extraite de spiruline enrichie en silicium selon un procédé selon l'invention.

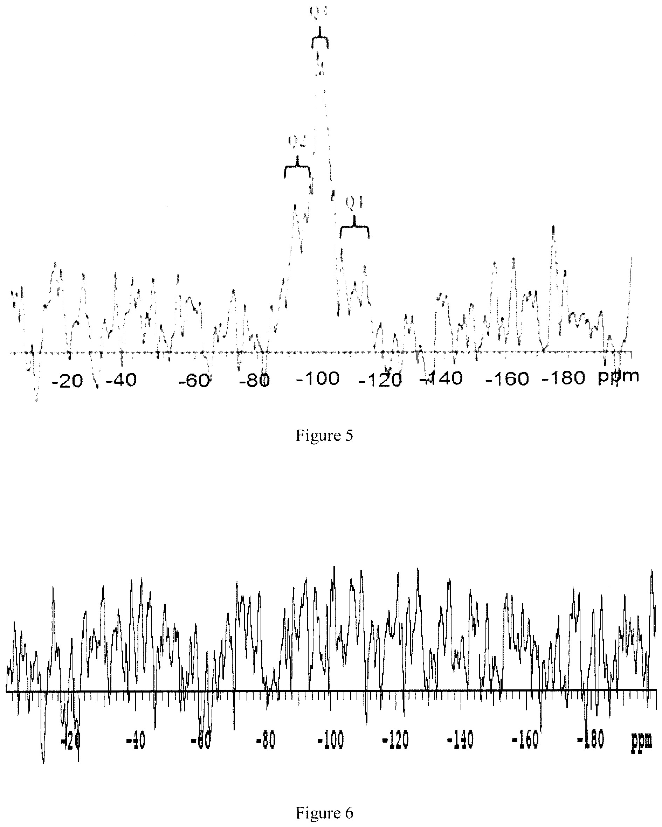

La phycocyanine est une protéine représentant 15 à 25% des protéines de la spiruline. Son extraction nécessite les étapes suivantes : broyage de la spiruline, centrifugation du broyat, récupération du surnageant, concentration de la phycocyanine par ultrafiltration tangentielle et lyophilisation du concentré. La poudre ainsi obtenue est totalement exempte de silicium résiduel sous forme minérale. Si des signaux RMN correspondant au silicium sont détectés en analysant la phycocyanine, cela signifie que le silicium a bien été métabolisé. La poudre est analysée en RMN du solide ²⁹Si. Le spectre obtenu est représenté dans la Figure 5. Interprétation :
- Des signaux correspondant au silicium sont détectés entre -90 et -115ppm. Ceci confirme la présence de silicium organique associé à la phycocyanine.
- Ces signaux ne correspondent pas aux signaux obtenus pour le metasilicate de sodium à - 68ppm. Il n'y a donc pas présence de résidus de métasilicate de sodium associé à la phycocyanine.

Structures chimiques correspondantes aux signaux détectés.
- L'entité Q4 correspond au déplacement chimique de -110à -115ppm environ. Les signaux obtenus selon cette structure sont groupés en un massif caractéristique.

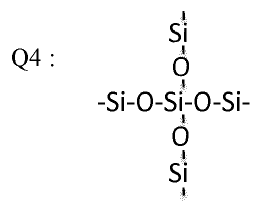

Cette structure correspond à de la silice dite « massive » fortement polymérisée contenant du silicium peu ou pas soluble.

Dans le spectre, ce signal est pratiquement du même ordre que le bruit de fond. L'entité Q4 est donc absente confirmant ainsi qu'il ne s'agit pas de silice « massive».
- L'entité Q3 correspond au déplacement chimique à - 100ppm environ. Les signaux obtenus avec cette structure sont groupés en un massif caractéristique.

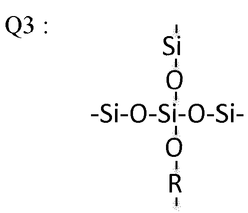

R représente un atome autre que le silicium.

Cette structure correspond à de la silice dite non « massive » car contenant du silicium lié par une liaison à des structures cellulaires, ce silicium est potentiellement soluble.
- L'entité Q2 correspond au déplacement chimique à - 90ppm environ : 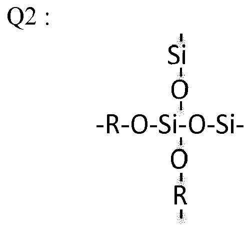 R représente un atome autre que le silicium.

Cette structure correspond à de la silice dite non « massive » car contenant du silicium lié par deux liaisons à des structures cellulaires, ce silicium est potentiellement plus soluble que l'entité Q3.

Les entités Q3 et Q2 sont présentes dans la phycocyanine extraite de spiruline enrichie en silicium selon le procédé. Il y a donc bien présence de silicium sous forme organique.

### 3. Analyse RMN du solide ²⁹Si de la phycocyanine extraite de spiruline enrichie en silicium sans le procédé

La phycocyanine est extraite selon le même procédé que précédemment mais à partir de spiruline cultivé dans un milieu de culture contenant du silicium qui n'a pas été apporté selon le procédé, mais a été apporté sous forme de poudre de métasilicate de sodium versée directement dans le milieu de culture sans avoir été préalablement dissous en présence de polyphénols selon le procédé. Le silicium solubilisé va rapidement polymériser et ne plus être disponible pour être absorbé par la spiruline.

Le spectre obtenu est représenté dans la figure 6.

Interprétation : Les signaux RMN entre -90 et -110ppm sont absents, les entités Q4, Q3 et Q2 sont donc absentes. Le silicium n'a pas été métabolisé et n'est pas associé à la phycocyanine.

### BIBLIOGRAPHIE

- Seaborne C.D. et al, 1993, Silicon: a nutritional beneficience for bones, brains and blood vessels?, Nutrition Today, Vol28, pp13-18.
- Refitt et al., 2003, Orthosilicic acid stimulates collagen type I synthesis and osteoblastic differentiation in human osteoblast-like cells in vitro, Bone 32, pp127-135.
- Schwarz K., 1977, Silicon, fiber and atherosclerosis, Lancet, Vol 26, pp 454-457.
- Schwarz K. et al, 1979, Inverse relation of silicon in drinking water and atherosclerosis in Finland, Lancet, Vol 5:1(8010), pp538-539.
- Loeper et al, 1979, The antiatheromatous action of silicon. Atherosclerosis, Vol 33, pp 497-308
- Maehira et al, 2011, Soluble silica and coral sand suppress high blood pressure and improve the related aortic gene expressions in spontaneously hypertensive rats, Nutr. Res., Vol 31(2), pp 147-156.
- Refitt et al., 1999, Silicic acid: its gastrointestinal uptake and urinary excretion in man and effects on aluminium excretion, journal of Inorganic Chemistry, Vol76, pp141-147.
- The EFSA Journal, 2004, Opinion of the scientific panel on dietetic products, nutrition and allergies on a request from the commission related to the tolerable upper intake level ofsilicon, 60, pp1-11.
- Puyfoulhoux G., Rouanet JM., Besançon P., Baroux B., Baccou JC., and Caporiccio B., 2001, Iron availability from iron-fortified spirulina by an in-vitro digestion/Caco-2 cell culture model, J. Agric. Food chem., Vol 49, pp1625-1629.
- Cases J., Puig M., Caporiccio B., Baroux B., Baccou JC., Besançon P. and Rouanet JM., 1999, Glutathione related enzymic activities in rats receiving high cholesterol or standard diets supplemented with two forms of selenium, Food Chemistry, Vol 65, pp 207-2211.
- Cases J., Wysocka I.A., Caporiccio B., Jouy N., Besançon P., Szpunar J. and Rouanet JM., 2002, Assessment of selenium bioavailability from high selenium spirulina subfractions in selenium deficient rats, J. Agric. Food Chem., Vol 50, pp 3867-3873.

## Revendications

1. Préparation de microalgue enrichie en silicium sous une forme soluble dans l'eau, ***caractérisée en ce que*** la proportion de silicium total dans ladite préparation est comprise entre 0,05% et 10% de la masse sèche de microalgue, du silicium étant détectable dans le compartiment intracellulaire de ladite microalgue.

2. Préparation selon la revendication 1, ***caractérisée en ce que*** ladite microalgue est la spiruline.

3. Préparation selon l'une des revendications 1 ou 2, ***caractérisée en ce que*** la proportion de silicium sous une forme soluble dans l'eau est comprise entre 5% et 90%, du silicium total présent dans ladite préparation.

4. Préparation selon l'une des revendications précédentes, ***caractérisée en ce que*** la proportion de silicium total dans ladite préparation est comprise entre 0,9% et 1,5% de la masse sèche de préparation, et la proportion de silicium sous une forme soluble dans l'eau est comprise entre 40% et 60% du silicium total.

5. Préparation selon l'une des revendications précédentes, ***caractérisée en ce que*** le silicium soluble présent dans ladite préparation de microalgue est détectable i) dans le compartiment intracellulaire et ii) associé aux membranes et/ou à la surface de ladite microalgue.

6. Préparation selon l'une des revendications précédentes, ***caractérisée en ce que*** le silicium soluble présent dans ladite préparation est détectable sous forme complexée à une protéine choisie parmi les phycobiliprotéines, les protéines membranaires, les protéines de structure.

7. Procédé de production d'une préparation de microalgue enrichie en silicium sous une forme soluble dans l'eau selon l'une des revendications 1 à 6, ***caractérisée en ce qu'**il* comprend les étapes suivantes :
a) la mise en culture de ladite microalgue dans des conditions et un milieu de culture adaptés jusqu'à l'obtention d'une culture de microalgues dont la biomasse sèche est comprise entre 0,1 g/l et 2 g/l de culture,
b) la préparation d'une solution aqueuse de silicium,
c) l'addition de la solution de silicium préparée en b) à la culture de microalgues obtenue en a),
d) la culture des microalgues sous agitation pendant une durée d'au moins 12 heures,
e) la récolte d'au moins une partie de la culture de microalgues.

8. Procédé selon la revendication précédente, ***caractérisé en ce que*** l'étape b) comprend la préparation d'une solution aqueuse de silicium et de polyphénols, par l'addition de silicium à une solution aqueuse de polyphénols, suivie d'un mélange par agitation.

9. Procédé selon la revendication 7 ou 8, ***caractérisé en ce qu'***à l'étape b) le silicium est ajouté sous une forme choisie dans le groupe constitué par : du métasilicate de sodium, du métasilicate de potassium une composition contenant du silicium dont tout ou partie est sous forme d'acide silicique ou un amendement silicique utilisé en agriculture.

10. Procédé selon l'une des revendications 7 à 9, dans lequel la concentration en silicium à l'issue de l'étape c) est comprise entre 0,5 et 4 g/litre de culture de microalgues.

11. Procédé selon l'une des revendications 8 à 10, dans lequel la concentration en polyphénols à l'issue de l'étape c) est comprise entre 5 et 60 mg/litre de culture de microalgues.

12. Procédé selon l'une des revendications 8 à 11, ***caractérisé en ce que*** la solution aqueuse de polyphénols consiste en un extrait de polyphénols naturels choisi dans le groupe constitué par : un extrait de polyphénols de plantes, un extrait de polyphénols d'olives, un extrait de polyphénols de feuilles de thé, un extrait de polyphénols de raisins, ou en un mélange de ces extraits.

13. Procédé selon l'une des revendications 7 à 12, dans laquelle à l'étape e), les microalgues sont récoltées par tamisage, centrifugation ou floculation, puis sont partiellement ou totalement déshydratées.

14. Composition comprenant une préparation de microalgue enrichie en silicium sous une forme soluble dans l'eau selon l'une des revendications 1 à 6 ou susceptible d'être obtenue par un procédé selon l'une des revendications 7 à 13, et au moins un composé choisi parmi : un excipient, un additif artificiel, un additif naturel, au moins un principe actif, un adjuvant, ou un mélange de ceux-ci.

15. Utilisation d'une composition comprenant une microalgue enrichie en silicium sous une forme soluble dans l'eau selon l'une des revendications 1 à 6, ou susceptible d'être obtenue par la mise en oeuvre d'un procédé selon l'une des revendications 7 à 13, pour la préparation d'une composition pharmaceutique, d'une composition cosmétique, d'une composition alimentaire ou d'un complément alimentaire.

## Patentansprüche

1. Mikroalgenzubereitung, die an Silicium in einer wasserlöslichen Form angereichert ist, ***dadurch gekennzeichnet, dass*** der Anteil an Gesamtsilicium in der Zubereitung im Bereich von 0,05 % bis 10 % der Mikroalgentrockenmasse liegt, wobei Silicium im intrazellulären Kompartiment der Mikroalge nachweisbar ist.

2. Zubereitung nach Anspruch 1, ***dadurch gekennzeichnet, dass*** es sich bei der Mikroalge um Spirulin handelt.

3. Zubereitung nach einem der Ansprüche 1 oder 2, ***dadurch gekennzeichnet, dass*** der Anteil an Silicium, welches in einer wasserlöslichen Form vorliegt, im Bereich von 5 % bis 90 % des Gesamtsiliciums liegt, welches in der Zubereitung vorliegt.

4. Zubereitung nach einem der vorhergehenden Ansprüche, ***dadurch gekennzeichnet, dass*** der Anteil an Gesamtsilicium, welches in der Zubereitung vorliegt, im Bereich von 0,9 % bis 1,5 % der Trockenmasse der Zubereitung liegt, wobei der Anteil an Silicium, welches in einer wasserlöslichen Form vorliegt, im Bereich von 40 % bis 60 % des Gesamtsiliciums liegt.

5. Zubereitung nach einem der vorhergehenden Ansprüche, ***dadurch gekennzeichnet, dass*** das lösliche Silicium, welches in der Mikroalgenzubereitung vorliegt, i) im intrazellulären Kompartiment und ii) derart, dass es an die Membranen und/oder die Oberfläche der Mikroalge gebunden ist, nachweisbar ist.

6. Zubereitung nach einem der vorhergehenden Ansprüche, ***dadurch gekennzeichnet, dass*** das lösliche Silicium, welches in der Zubereitung vorliegt, derart nachweisbar ist, dass es in Komplexbindung mit einem Protein vorliegt, welches aus den Phycobiliproteinen, den Membranproteinen, den Strukturproteinen ausgewählt ist.

7. Verfahren zur Herstellung einer Mikroalgenzubereitung, die an Silicium in einer wasserlöslichen Form angereichert ist, nach einem der Ansprüche 1 bis 6, ***dadurch gekennzeichnet, dass*** es die folgenden Schritte umfasst:
a) Anzüchten der Mikroalge unter geeigneten Bedingungen und in einem geeigneten Kulturmedium, bis eine Mikroalgenkultur erhalten wird, deren Trockenbiomasse im Bereich von 0,1 g/L bis 2 g/L an Kultur liegt.
b) Herstellen einer wässrigen Siliciumlösung,
c) Hinzufügen der unter b) hergestellten Siliciumlösung zur Mikroalgenkultur, die unter a) erhalten wurde,
d) Anzüchten der Mikroalgen unter Rühren während einer Dauer von mindestens 12 Stunden,
e) Entnehmen mindestens eines Teils der Mikroalgenkultur.

8. Verfahren nach dem vorhergehenden Anspruch, ***dadurch gekennzeichnet, dass*** der Schritt b) das Herstellen einer wässrigen Lösung von Silicium und von Polyphenolen umfasst, indem Silicium einer wässrigen Lösung von Polyphenolen zugesetzt wird, woraufhin ein Vermischen durch Rühren erfolgt.

9. Verfahren nach Anspruch 7 oder 8, ***dadurch gekennzeichnet, dass*** in Schritt b) das Silicium in einer Form zugesetzt wird, die aus der Gruppen ausgewählt ist, welche aus den folgenden besteht: Natriummetasilicat, Kaliummetasilicat, einer Zusammensetzung, die Silicium enthält, wobei dieses vollständig oder teilweise in Form von Kieselsäure vorliegt, oder einem siliciumhaltigen Bodenverbesserer, der in der Landwirtschaft verwendet wird.

10. Verfahren nach einem der Ansprüche 7 bis 9, wobei die Konzentration an Silicium nach Abschluss des Schrittes c) im Bereich von 0,5 bis 4 g/Liter an Mikroalgenkultur liegt.

11. Verfahren nach einem der Ansprüche 8 bis 10, wobei die Konzentration an Polyphenolen nach Abschluss des Schrittes c) im Bereich von 5 bis 60 mg/Liter an Mikroalgenkultur liegt.

12. Verfahren nach einem der Ansprüche 8 bis 11, **dadurch gekennzeichnet, dass** die wässrige Lösung von Polyphenolen aus einem Extrakt natürlicher Polyphenole besteht, der aus der Gruppe ausgewählt ist, welche aus den folgenden besteht: einem Extrakt von pflanzlichen Polyphenolen, einem Extrakt von Olivenpolyphenolen, einem Extrakt von Teeblätterpolyphenolen, einem Extrakt von Traubenpolyphenolen oder einer Mischung dieser Extrakte.

13. Verfahren nach einem der Ansprüche 7 bis 12, wobei im Schritt e) die Mikroalgen durch Sieben, Zentrifugation oder Flockung entnommen werden, woraufhin ihnen teilweise oder vollständig das Wasser entzogen wird.

14. Zusammensetzung, die eine an Silicium in einer wasserlöslichen Form angereicherte Mikroalgenzubereitung umfasst, welche den Ansprüchen 1 bis 6 entspricht oder mittels eines Verfahrens nach einem der Ansprüche 7 bis 13 erhalten werden kann, sowie mindestens eine Verbindung, die aus den folgenden ausgewählt ist: einem Trägerstoff, einem künstlichen Zusatzstoff, einem natürlichen Zusatzstoff, mindestens einem Wirkstoff, einem Hilfsstoff oder einer Mischung davon.

15. Verwendung einer Zusammensetzung, die eine an Silicium in einer wasserlöslichen Form angereicherte Mikroalge umfasst, welche den Ansprüchen 1 bis 6 entspricht oder erhalten werden kann, indem ein Verfahren nach einem der Ansprüche 7 bis 13 durchgeführt wird, zur Herstellung einer pharmazeutischen Zusammensetzung, einer kosmetischen Zusammensetzung, einer Nahrungsmittelzusammensetzung oder eines Nahrungsergänzungsmittels.

## Claims

1. A microalgae preparation that is enriched in silicon in a water-soluble form, ***characterised in that*** the proportion of total silicon in said preparation is between 0.05 % and 10 % of the dry mass of microalgae, silicon being detectable in the intracellular compartment of said microalgae.

2. The preparation according to claim 1, ***characterised in that*** said microalgae is spirulina.

3. The preparation according to one of claims 1 or 2, ***characterised in that*** the proportion of silicon in a water-soluble form is between 5 % and 90 % of the total silicon that is present in said preparation.

4. The preparation according to one of the above claims, ***characterised in that*** the proportion of total silicon in said preparation is between 0.9 % and 1.5 % of the dry mass of the preparation, and the proportion of silicon in a water-soluble form is between 40 % and 60 % of the total silicon.

5. The preparation according to one of the above claims, ***characterised in that*** the soluble silicon which is present in said microalgae preparation is detectable i) in the intracellular compartment and ii) associated with the membranes and/or the surface of said microalgae.

6. The preparation according to one of the above claims, ***characterised in that*** the soluble silicon which is present in said preparation is detectable in complexed form with a protein selected from phycobiliproteins, membrane proteins, structural proteins.

7. A process for producing microalgae preparation that is enriched in silicon in a water-soluble form according to one of claims 1 to 6, ***characterised in that*** it comprises the following steps:
a) culturing the said microalgae under suitable conditions and in a suitable culture medium until a microalgae culture is obtained which has a dry biomass between 0.1 g/L and 2 g/L of culture,
b) preparing an aqueous solution of silicon,
c) adding the silicon solution prepared in b) to the microalgae culture obtained in a),
d) culturing the microalgae under agitation for at least 12 hours,
e) harvesting the microalgae culture at least partially.

8. The process according to the above claim, ***characterised in that*** step b) comprises preparing an aqueous solution of silicon and polyphenols, by adding silicon to an aqueous solution of polyphenols, and then mixing by stirring.

9. The process according to claim 7 or 8, ***characterised in that*** in step b) said silicon is added in a form selected from the group consisting of: sodium metasilicate, potassium metasilicate, a composition containing silicon which is entirely or partially in the form of silicic acid, or a silicic soil amendment used in agriculture.

10. The process according to one of claims 7 to 9, wherein the silicon concentration at the end of step c) is between 0.5 and 4 g/litre of microalgae culture.

11. The process according to one of claims 8 to 10, wherein the polyphenol concentration at the end of step c) is between 5 and 60 mg/litre of microalgae culture.

12. The process according to one of claims 8 to 11, ***characterised in that*** the aqueous polyphenol solution consists of an extract of natural polyphenols selected from the group consisting of: a plant polyphenol extract, an olive polyphenol extract, a tea leaf polyphenol extract, a grape polyphenol extract, or a mixture of these extracts.

13. The process according to one of claims 7 to 12, wherein in step e), the microalgae are collected by sieving, centrifugation or flocculation, and then partially or completely dehydrated.

14. A composition comprising a microalgae preparation which is enriched in silicon a water-soluble form according to one of claims 1 to 6 or obtainable by a process according to one of claims 7 to 13, and at least one compound selected from: an excipient, an artificial additive, a natural additive, at least one active ingredient, an adjuvant, or a mixture thereof.

15. Use of a composition comprising a microalgae enriched in silicon in a water-soluble form according to one of claims 1 to 6, or obtainable by carrying out a process according to one of claims 7 to 13, for making of a pharmaceutical composition, a cosmetic composition, a food composition or a food supplement.
